# EUROPEAN PATENT APPLICATION

(11) **EP 2 659 864 A1**
(43) Date of publication of application: **06.11.2013**
(21) Application number: 13165839.5
(22) Date of filing: 29.04.2013
(51) Int. Cl.: A61F 13/00

(54) **Systems for Providing Wound Care Using Air Fluidized Therapy**

(30) Priority: 03.05.2012 US 201261641983 P
(71) Applicant: Hill-Rom Services, Inc., Batesville, IN 47006-9167 (US)
(72) Inventor: Howell, Charles, A, Batesville, IN Indiana 47006 (US); Lachenbruch, Charles A., Batesville, IN Indiana 78734 (US)
(74) Representative: Findlay, Alice Rosemary

(57) **Abstract**

A system for wound care using air fluidized therapy is disclosed. The system comprises a wound care device with a reservoir of beads and a filter sheet contains the beads within the reservoir while contacting the user's skin during use. Air flow through the reservoir of beads fluidizes the beads during use. The wound care device is equipped with a quick disconnect coupling and can be separated from the fluid line and disposed off.

## Description

Preventing and managing wounds is a significant concern for caregivers and patients alike. Appropriate drainage of the fluids secreted at wound sites, temperature management and effective pressure distribution of at risk and/or a wound site are ongoing challenges. While several systems and methods exist for prevention and/or management of a wound, a need exists for continued development in this area.

The present disclosure includes one or more of the features recited in the appended claims and/or the following features which, alone or in any combination, may comprise patentable subject matter.

One embodiment of a system for providing wound care comprises a disposable wound care device comprising beads encapsulated in a reservoir. The beads are configured to be separated from a user's skin by a fluid permeable, bead impermeable filter sheet. A fluid supply device is configured to supply air to the reservoir while a control interface is configured to enable a user to regulate the properties of the fluid supplied to the reservoir.

Another embodiment of a disposable wound care device configured to be placed on a user's skin comprises a reservoir encapsulating beads and a fluid permeable, bead impermeable filter sheet. A first side of the filter sheet is configured to be in contact with a user's skin while a second side forms a portion of the reservoir. At least one valve is configured to selectively retain fluid in the reservoir.

Another embodiment of a system for providing wound care comprises wound care device comprising beads encapsulated in a reservoir, the wound care device configured to attach to a user's body part. The beads are configured to be separated from a user's skin by a fluid permeable, bead impermeable filter sheet. A fluid supply device is configured to supply air to the reservoir while a control interface is configured to enable a user to regulate the properties of the fluid supplied to the reservoir.

The invention will now be further described by way of example with reference to the accompanying drawings, in which:

FIG. 1 is a perspective view of a system for providing wound care, constructed according to one or more of the principles disclosed herein;

FIG. 2 is a perspective view of another system for providing wound care, constructed according to one or more of the principles disclosed herein;

FIG. 3 is a perspective view of another system for providing wound care , constructed according to one or more of the principles disclosed herein;

FIG. 4A & FIG. 4B are perspective and cross-sectional views respectively of a wound care device, for use with a system for providing wound care, constructed according to one or more of the principles disclosed herein;

FIG. 5A & FIG. 5B are perspective and cross-sectional views respectively of another wound care device, for use with a system for providing wound care, constructed according to one or more of the principles disclosed herein;

FIG. 6 is a perspective view of a system for providing wound care, constructed according to one or more of the principles disclosed herein;

FIG. 7A & FIG. 7B are perspective and cross-sectional views respectively of a wound care device for use with a system for providing wound care shown in FIG. 6, constructed according to one or more of the principles disclosed herein.

The embodiments of the claimed subject mater and the various features and advantageous details thereof are explained more fully with reference to the non-limiting embodiments and examples that are described and/or illustrated in the accompanying drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be briefly mentioned or omitted so as to not unnecessarily obscure the embodiments of the claimed subject matter described. The examples used herein are intended merely to facilitate an understanding of ways in which the claimed subject matter may be practiced and to further enable those of skill in the art to practice the embodiments of the claimed subject matter described herein. Accordingly, the examples and embodiments herein are merely illustrative. Moreover, it is noted that like reference numerals represent similar parts throughout the several views of the drawings.

It is understood that the subject matter claimed is not limited to the particular methodology, protocols, devices, apparatus, materials, applications, etc., described herein, as these may vary. It is also to be understood that the terminology used herein is used for the purpose of describing particular embodiments only.

Unless defined otherwise, all technical and scientific terms used herein have the same meanings as commonly understood by one of ordinary skill in the art.

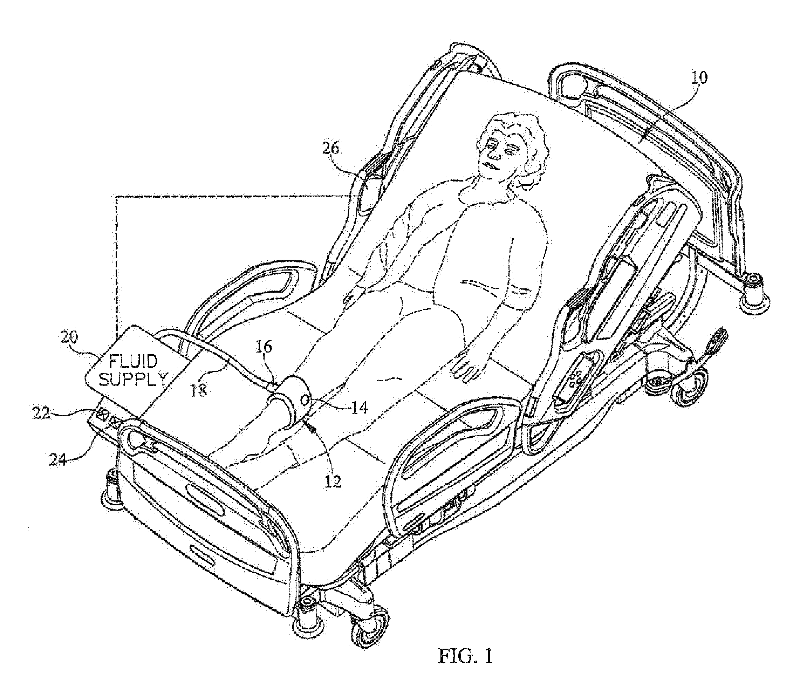

One embodiment of a system for providing wound care is shown in FIG. 1. As shown in FIG.1 a wound care device 12 is supplied fluid from a fluid supply device 20 through a fluid line 18. A valve 14 mounted on the wound care device 12 is configured to adjustably meter the amount of fluid that is vented out of the wound care device 12 to the surrounding atmosphere. The valve 14 may be of any type including but not limited to a check valve or a metering valve. The fluid supply device 20 is a blower in this embodiment while in other embodiments the fluid supply device may be a compressor, a fan or any other device capable of supplying fluid. As shown in FIG. 1 the fluid supply device in this embodiment is mounted on a patient support apparatus 10. The fluid supply device 20 in this embodiment is used for other operations of the bed such as providing fluid to inflate a mattress situated on the patient support apparatus 10. The wound care device 12 is configured to be disconnected from the fluid line 18 by a quick disconnect coupling 16 in this embodiment. In another embodiment, any type of coupling may be used to disconnect the wound care device 12 from the fluid line 18. At least one temperature sensor 24 monitors the temperature of fluid supplied by the fluid supply device 20 to the wound care device 12. At least one pressure sensor 22 monitors the pressure of the fluid supplied by the fluid supply device 20 to the wound care device 12 in this embodiment. In another embodiment the back pressure of the supplied fluid may be used to determine pressure of the fluid supplied. The fluid supply device 20 is controlled by a patient support apparatus control interface 26 via an electrical signal in this embodiment. The patient support apparatus control interface 26 comprises at least one processor which provides the control signals. In other embodiments the patient support apparatus control interface 26 may communicate with the fluid supply device 20 using any type of signal including but not limited to optical or radio frequency signals. In this embodiment patient support apparatus control interface 26 is a graphical control interface for operating the patient support apparatus 10. The patient support apparatus control interface 26 allows caregiver input to regulate the properties of the supplied fluid. Such properties include temperature, volumetric flow rate and pressure values and/or profiles (time dependence) of the aforementioned parameters of the fluid supplied by the fluid supply device 20. In this embodiment the patient support apparatus control interface 26 also comprises a display which allows for display of information to the caregiver including but not limited to the duration and/or profile (time dependence) of temperature and/ or volumetric flow rate and/ or pressure of the fluid supplied to the wound care device 12. In this embodiment, the information is presented to the caregiver in a graphical form while in other embodiments the information may be presented in any form including but not limited to text or audio signals.

The system for providing wound care as shown in FIG. 1 shows a wound care device 12 configured to be wrapped around a user's limb. The wound care device 12 is ideally used for prevention of skin breakdown and/or to facilitate healing of a wound. Varying the skin temperature at the site of skin susceptible to breakdown and/or wound site may be desirable to help in prevention and /or promote healing. The fluid supply device 20 in the system for providing wound care as shown in FIG. 1 comprises the ability to vary the temperature of fluid being supplied to the wound care device 12. In this embodiment a thermoelectric heating and cooling unit is integral to the fluid supply device and is controlled by the patient support apparatus control interface 26. In other embodiments, any other means of heating and cooling the fluid supplied to the wound care device 12 may be used. The fluid supplied to the wound care device 12 is varied in temperature and/or flow rate to achieve up to 500 Watt per square meter of energy dissipation or cooling of the patient's skin in contact with the wound care device 12 in one embodiment. In another embodiment the temperature of fluid supplied to the patient's skin may be as high as 45 degrees Celsius.

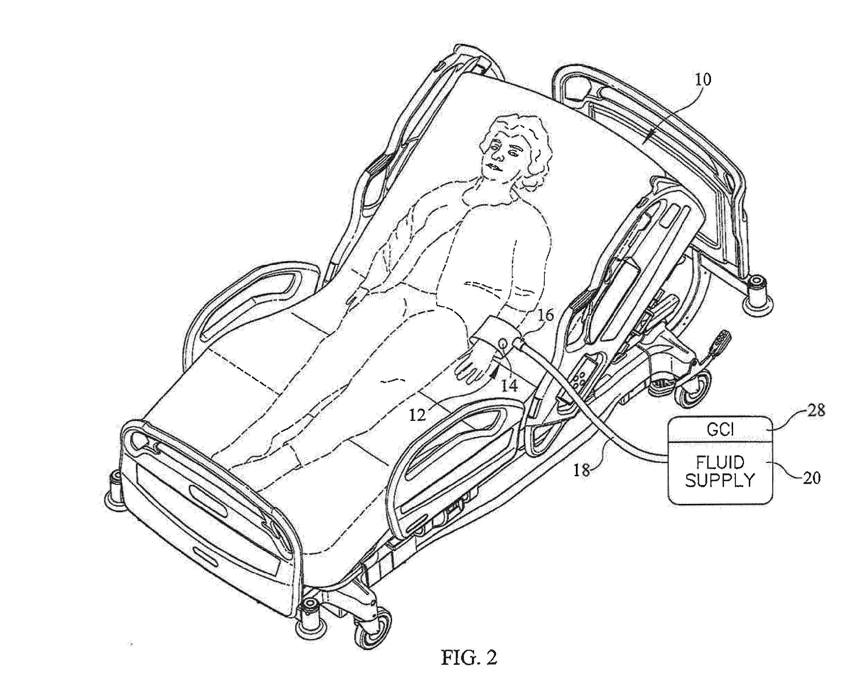

FIG.2 shows an embodiment of the system for providing wound care wherein the system has a dedicated control interface 28. In the embodiment shown in FIG. 2, the fluid supply device is a standalone unit with the control interface 28 built in. In another embodiment the control interface is detachable from the control interface. In yet another embodiment the control interface 28 is remotely located and communicates with the fluid supply device via a communication network.

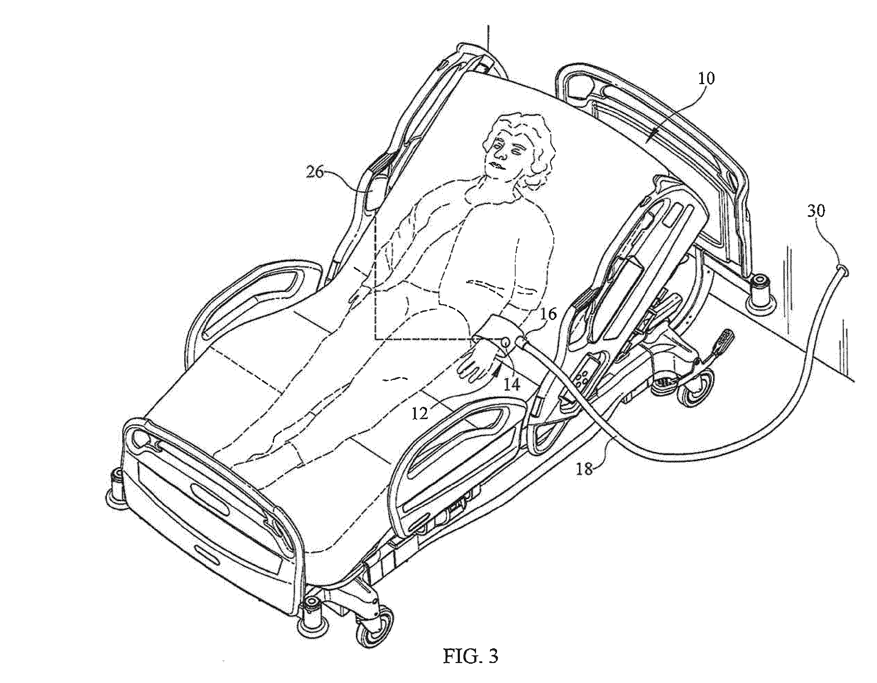

FIG.3 shows an embodiment of the system for providing wound care wherein the wound care device 12 receives fluid from the hospital air supply 30. The patient support apparatus control interface 26 controls the valve 14 to regulate the pressure and/or air flow rate in to and/or through the wound care device 12 by venting air from the wound care device to the surrounding atmosphere.

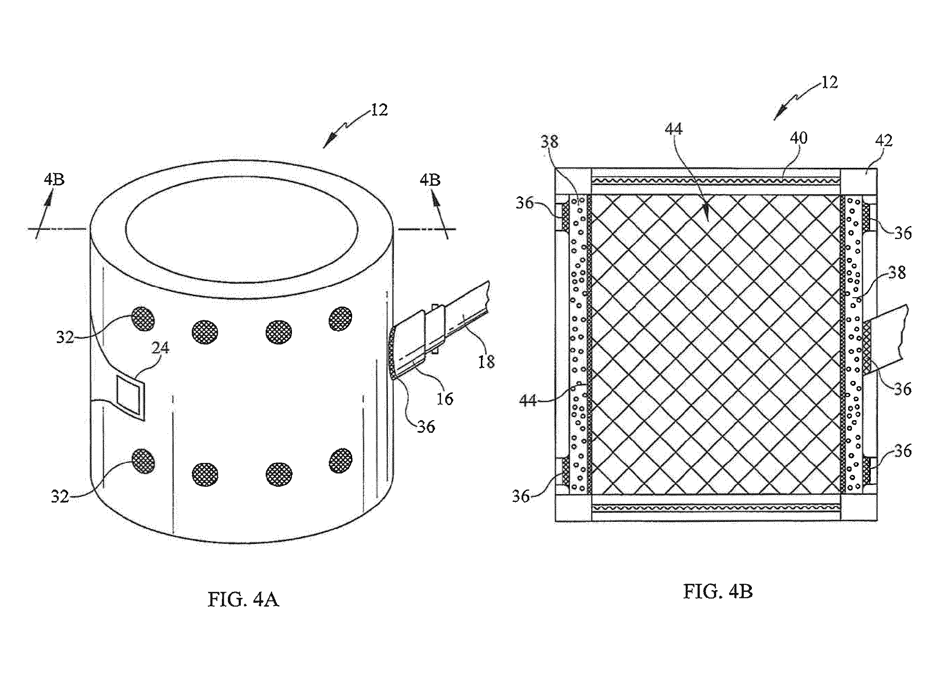

FIG 4A & FIG.4B show one embodiment of the wound care device 12 for use with the system for providing wound care. The figures show a wound care device 12 configured to be wrapped around a user's limb. FIG. 4B is a cross-sectional view of FIG. 4A. Wound care device 12 comprises an inner filter sheet 44, which is in contact with the user's skin when the wound care device 12 is used. The filter sheet 44 forms part of the reservoir of beads 38 and its construction is such that the filter sheet 44 is impermeable by the beads yet it allows transfer of fluids through the sheet. Filter sheet 44 is made of a meshed Polyester yarn with 70 denier yarn size and 30-40 micron mesh opening in this embodiment. The filter sheet 44 also comprises healing factors including but not limited to oxidizing species, Hydrogen Peroxide and growth factors such as Vascular Endothelial Growth Factor (VEGF) to promote wound healing. The growth factors are incorporated into the filter sheet as a coating in this embodiment, in other embodiments the growth factors may be incorporated into the fabric material of the filter sheet. In another embodiment the filter sheet 44 may be made of any material and construction. The beads are medical grade microspheres, 50-150 micron diameter with a hygroscopic coating in this embodiment. In another embodiment the beads may be of any size, shape and with any type of coating. The filter sheet 44 is thin and pliable so as to allow the effect of the impingement of the beads on the filter sheet 44 to be felt by the skin of the user. The reservoir 38 is encompassed on other sides by an air and bead impermeable fabric 42. The reservoir 38 receives air from a fluid line 18 configured to be coupled to the wound care device 12 by the quick disconnect coupling 16 mounted on the air and bead impermeable fabric 42. Bead impermeable, air permeable filter 36 between the reservoir of beads and the quick disconnect connector 16 ensures that the beads do not flow in to the fluid supply device 20 or the supplied fluid via the fluid line 18. Filter 36 also ensures that a used wound care device can be disposed of using the quick disconnect coupling 16 without contaminating the fluid line 18 with biologic material. In this embodiment the air and bead impermeable fabric 42 comprise air vents 32 which allow air to flow out of the reservoir of beads 38. Filters 36 are attached at the interface of the air vents 32 and the reservoir of beads 38 so that the beads are contained inside the reservoir of beads 38. In other embodiments such as the embodiment shown in FIG. 1, a valve 14 is incorporated in the air and bead impermeable fabric 42 to allow the ability to meter air flowing out of the wound care device 12. In this embodiment, sealing strip 40 is placed on either end of the filter sheet 44 so that air does not leak out between the interface of the skin and the sealing strip 40. Sealing strip 40 is of an elastomeric material in this embodiment while in another embodiment may be of any material including but not limited to an adhesive compound. In another embodiment, sealing strip 40 is not included in the construction of the wound care device 12. The wound care device 12 is held in place wrapped around a patient's limb in this embodiment using a hook and loop connection 24. Any other type of connection may be used to hold the wound care device in place in other embodiments, including, but not limited to an adhesive connection and/or a vacuum connection. In addition, the wound care device may be constructed in the form of a sleeve or collar having a suitable snug fit relative to a patient's limb.

During operation of the wound care device 12 shown in FIG. 4A & FIG. 4B, air flows in through the fluid line 18 in to the reservoir of beads 38 via the quick disconnect coupling 16 and the filter 36. Air flows out of the reservoir of beads to the surrounding atmosphere through the filters 36 and the air vents 32. During operation, the pressure in the reservoir of beads 38 is higher than the atmospheric pressure and the pressure with which air is supplied via the fluid line 18 is higher than the pressure in the reservoir of beads 38. The aforementioned flow of air causes agitation of beads within the reservoir and fluidizes the volume of beads. This fluidized flow of beads within the reservoir impinges on the skin of the user through the filter sheet 44 to provide stimulation to the skin of the user. When the wound care device is applied at the site of an open wound, biologic material such a blood and pus may flow through the fluid permeable filter sheet 44 and are absorbed and/or adsorbed by the coating of the beads. The wound care device is disposable. In other words the device is inexpensive enough that it is economically acceptable to discard the device after it has served for its effective life rather than re-use it or refurbish it. Another attribute that causes the device to be disposable is that it is small enough to be discarded by a caregiver in customarily available waste receptacles as opposed to requiring special effort and/or special handling and/or special assistance (and the associated costs) to remove the device from a health care facility. By contrast, a hospital bed would not be considered disposable because it is an expensive piece of capital equipment and its removal from the facility would require some amount of preplanning and the assistance of a mover equipped to handle objects that are heavy, bulky or both. Once the wound care device 12 has been used beyond its effective life, it is removed and replaced by a fresh wound care device 12. Determination that the wound care device 12 has exceeded its effective life is made by the patient support apparatus control interface 26 and/or control interface 28 based on an increase in back pressure sensed in fluid line 18 and/or pressure read by pressure sensor 22. This may be an indication that enough biologic material has been collected by the beads and clumps formed such that fluidization of the beads has been impacted. Visual inspection by the caregiver and/or calculation of duration of use of a particular wound care devices are other ways to determine end of the effective life of the wound care device 12 in other embodiments.

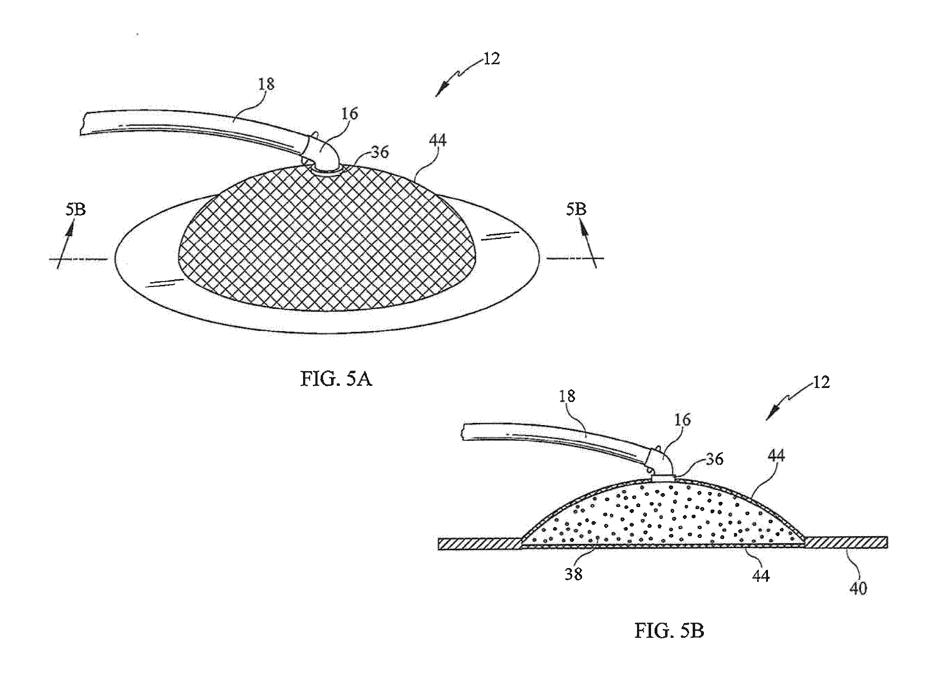

FIG 5A & FIG.5B show another embodiment of the wound care device 12 for use with the system for providing wound care. The figures show a wound care device 12 configured to be attached to a user's skin. Wound care device 12 comprises a filter sheet 44, a portion of which is in contact with the user's skin when the wound care device 12 is used. The filter sheet 44 forms the reservoir of beads 38 such that the filter sheet 44 is impermeable by the beads yet it allows transfer of fluids through the sheet. Air flows into the reservoir of beads 38 from the fluid line 18 via the quick disconnect connecter 16 and the filter 36 to fluidize the beads. The wound care device 12 is in the form of a patch which is placed over an area of the skin susceptible to skin breakdown and/or with a wound.

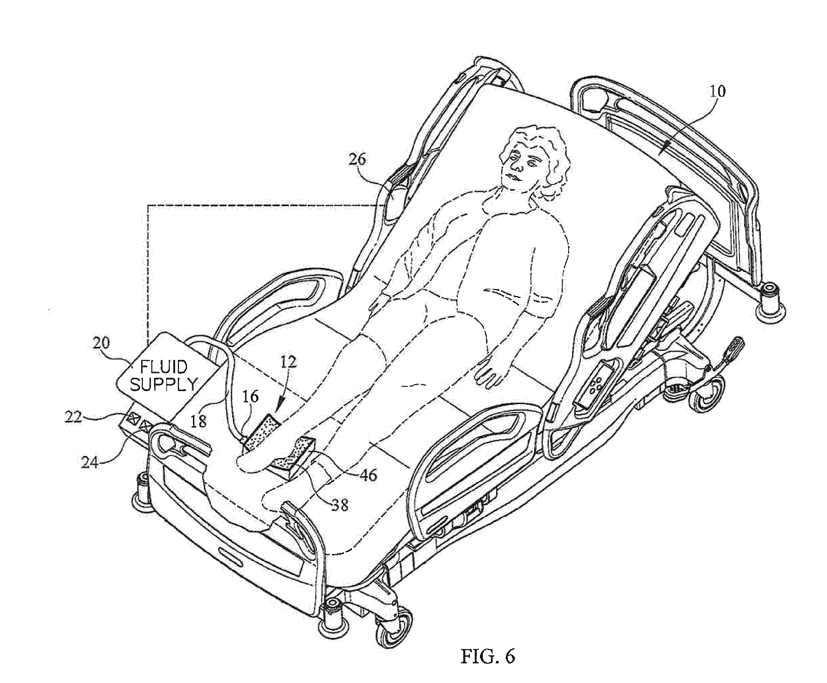

One embodiment of a system to provide wound care and an associated wound care device is shown in FIGS. 6, &A and 7B. The wound care device 12 in this embodiment is a cushion that supports at least a portion of the user's body when in operation such that the weight of the supported portion of the user's body is transmitted to the patient support apparatus 10 via the wound care device 12. This is in contrast to the wrap of FIGS. 4A and 4B and the patch of FIGS. 5A and 5B in that the cushion serves both a supporting function and a wound care function whereas the wrap and patch are fundamentally wound care devices that do not provide any meaningful support function. This is true even though loads may be transferred through the wrap and the patch, for example if the patch is attached to the buttocks of a patient who is lying on a mattress in a supine posture The wound care device 12 as shown in FIG. 6 comprises a container 46 forming a reservoir of beads 38.

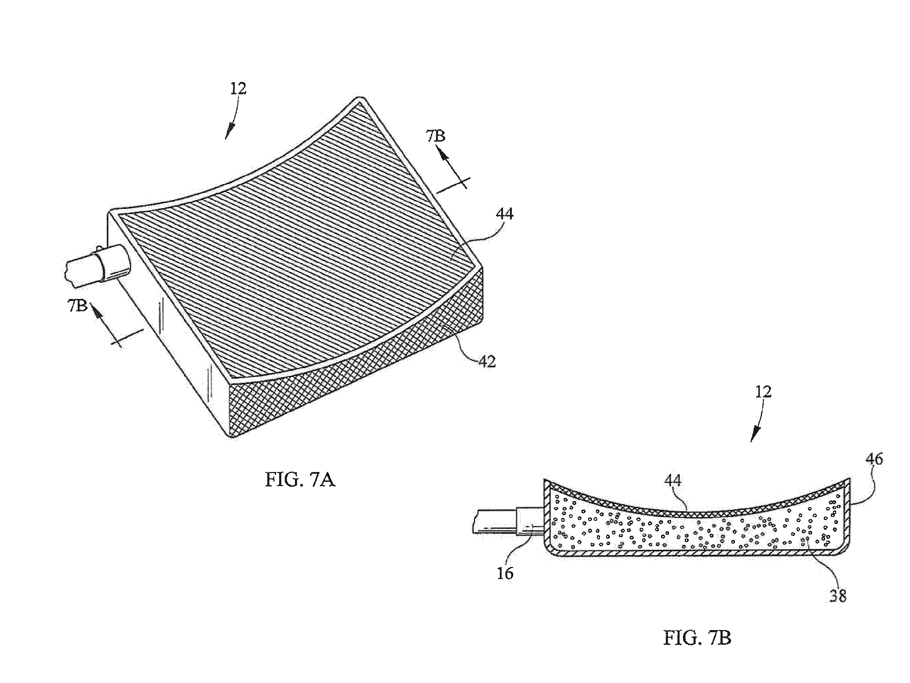

FIG. 7A & FIG. 7B show an embodiment of the wound care device 12 for use with a system to provide wound care shown in FIG. 6. The container 46 is made of a polymeric material in this embodiment and is substantially rigid in this embodiment. In another embodiment, the container may be made of any material including but not limited to composites and/or metal. The container 46 in this embodiment has a base and two side walls, while in another embodiment the container 46 may have any number of side walls. In this embodiment the face between the side walls of the container 46 is closed off by an air impermeable fabric 42 while the top of the container is closed off by the fluid permeable bead impermeable filter sheet 44. In another embodiment, the filter sheet 44 is used instead of the air and bead impermeable fabric 42 and attaches to the sides of the container 46 to form a reservoir of beads 38. A fluid line 18 supplies air to the reservoir of beads 38 through a quick disconnect coupling 16. A filter 36 is included at the interface of the fluid line 18 and the reservoir of beads 38 in another embodiment.

In this embodiment, the user places their limb between the side walls of the container 46 as shown in FIG. 6. Air flowing into the reservoir of beads 38 from the fluid line 18 fluidizes the beads and the user's limb is supported by the fluidized beads. Flow rate and/or pressure of air supplied by the fluid line 18 is variable and air with pressure of up to 25 mm of Hg may be provided to the reservoir of beads. Any part of the body may be supported by the reservoir of beads 38 including but not limited to the sacrum region, heels and elbows of the user. Extremities supported by air fluidized beads experience substantially less local shear force on the skin as opposed to the same extremity resting on a firm mattress or sheet.

The systems for providing wound care described in FIG. 1 through FIG. 7B are used for prevention and wound healing as described in the table below which is intended to be exemplary and non-limiting. Other embodiments may have other modalities not described below.

| Goal | Modality |
|---|---|
| Prevention of skin breakdown in areas susceptible to pressure ulcers | Tissue cooling to combat inflammation and progression of injury (Up to 500 Watt per sq. meter of energy dissipation from skin and /or lower skin temperature from low to mid 60 degree Fahrenheit) |
| Maintain intact skin surface | Prevent friction and abrasion injury by suspension in bead bath and minimizing shear forces on the skin immersed. (Air with pressure of up to 25 mm of Hg may be provided to the reservoir of beads) |
| Prevention of further skin breakdown for areas of skin afflicted with Stage I pressure ulcers | Tissue cooling to combat inflammation and progression of injury (Up to 500 Watt per sq. meter of energy dissipation from skin and /or lower skin temperature from low to mid 60 degree Fahrenheit) |
| Facilitate healing for open wounds with Stage II - IV pressure ulcers (Proliferative Phase) | Mild skin warming to increase perfusion and metabolically accelerate various healing processes. (Supply air with a temperature up to 45 degrees Celsius) |
| Evaporation of open wound exudate which can cause concentric expansion of wound as it moistens and breaks down tissue at periphery | Variable air flow rate to evaporate exudates but not excessively dry skin. |
| Promote collagen network's growth and strengthening for open wound areas with Stage II - IV pressure ulcers (Remodeling Phase) | Mechanical stimulation via bead impact, vibration and massage. Healing Factors applied to filter sheet. |

The use of the terms "a" and "an" and "the" and similar referents in the context of describing the subject matter (particularly in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. Furthermore, the foregoing description is for the purpose of illustration only, and not for the purpose of limitation. The use of any and all examples, or exemplary language (e.g., "such as") provided herein, is intended merely to better illustrate the subject matter. The use of the term "based on" and other like phrases indicating a condition for bringing about a result, both in the claims and in the written description, is not intended to foreclose any other conditions that bring about that result. No language in the specification should be construed as indicating any non-claimed element as essential to the practice of the invention as claimed.

Preferred embodiments are described herein, including the best mode known to the inventor for carrying out the claimed subject matter. Of course, variations of those preferred embodiments will become apparent to those of ordinary skill in the art upon reading the foregoing description. The inventor expects skilled artisans to employ such variations as appropriate. Moreover, any combination of the above-described elements in all possible variations thereof is encompassed unless otherwise indicated herein or otherwise clearly contradicted by context.

Embodiments of the invention can be described with reference to the following numbered clauses, with preferred features laid out in the dependent clauses:
1. A system for providing wound care, comprising:
   a disposable wound care device comprising beads encapsulated in a reservoir, said beads configured to be separated from a user's skin by a fluid permeable, bead impermeable filter sheet;
   a fluid supply configured to supply air to said reservoir;
   a control interface configured to communicate with said fluid supply.
2. The system for providing wound care of clause 1, wherein said disposable wound care device is configured to wrap around a user's body part.
3. The system for providing wound care of clause 1, wherein said disposable wound care device is a patch configured to be applied to the skin of a user.
4. The system for providing wound care of clause 1, further comprising a quick disconnect coupling between said disposable wound care device and said fluid supply.
5. The system for providing wound care of clause 1, wherein said fluid supply is mounted to a bed.
6. The system for providing wound care of clause 1, wherein said fluid supply is a dedicated unit.
7. The system of providing wound care of clause 1, wherein said fluid supply is a hospital air supply line.
8. The system of providing wound care of clause 1, wherein said control interface is a bed controller.
9. The system of providing wound care of clause 1, wherein said control interface is a dedicated unit.
10. The system of providing wound care of clause 1, further comprising a temperature sensor in communication with said control interface, said temperature sensor configured to indicate the temperature of fluid supplied by the fluid supply to said reservoir.
11. The system of providing wound care of clause1, wherein said fluid supply is configured to control the temperature of air supplied to said reservoir.
12. The system of providing wound care of clause 11, wherein said fluid supply is configured to carry away energy up to 500 Watts per square meter from a user's skin in contact with said filter sheet.
13. The system of providing wound care of clause 11, wherein said fluid supply is configured to supply air with a temperature up to 45 degrees Celsius.
14. The system of providing wound care of clause 1, wherein said fluid supply is configured to provide variable air flow to said reservoir.
15. The system of providing wound care of clause 1, further comprising at least one healing factor applied to said filter sheet.
16. The system of providing wound care of clause 1, wherein said fluid supply is configured to supply air with a pressure of up to 25 millimeters of Mercury.
17. A disposable wound care device configured to be placed on a user's skin, comprising:
   a reservoir encapsulating beads;
   a fluid permeable bead impermeable filter sheet, a first side of said filter sheet is configured to be in contact with a user's skin while a second side forms a portion of said reservoir;
   at least one valve configured to selectively retain fluid in said reservoir.
18. The disposable wound care device of clause 17 further comprising a fluid supply which is configured to supply a variable volume of fluid to said reservoir.
19. The disposable wound care device of clause 18 further comprising a control interface configured to provide control signals to said fluid supply.
20. The disposable wound care device of clause 19 wherein said control interface provides control signals to vary the temperature of the fluid supplied by said fluid supply to said reservoir.
21. The disposable wound care device of clause 17, further comprising at least one healing factor applied to said filter sheet.
22. A system for providing wound care, comprising:
   a wound care device comprising beads encapsulated in a reservoir, said beads configured to be separated from a user's skin by a fluid permeable, bead impermeable filter sheet, said wound care device configured to attach to a user's body part;
   a fluid supply configured to supply air to said reservoir;
   a control interface configured to communicate with said fluid supply.
23. The system for providing wound care of clause 22, wherein said wound care device wherein said wound care device is configured to wrap around a user's body part.
24. The system for providing wound care of clause 22, wherein said wound care device is a patch configured to be applied to the skin of a user.
25. A wound care device comprising a reservoir containing a fluidizable medium and defined at least in part by a gas permeable filter sheet intended to be in local contact with a user, the reservoir adapted to receive a stream of gas and to discharge at least some of the received gas through the filter sheet, the fluidizable medium being fluidizable by the stream of gas, the filter sheet allowing the user's skin to feel impingement of the fluidizable medium when the medium is fluidized.

## Claims

1. A system for providing wound care, comprising:
a disposable wound care device comprising beads encapsulated in a reservoir, said beads configured to be separated from a user's skin by a fluid permeable, bead impermeable filter sheet;
a fluid supply configured to supply air to said reservoir;
a control interface configured to regulate properties of the fluid.

2. The system of claim 1, wherein said disposable wound care device is configured to wrap around a user's body part.

3. The system of claim 1, wherein said disposable wound care device is a patch configured to be applied to the skin of a user.

4. The system of claim 3 wherein the patch is attachable to the user

5. The system of claim 2 or 3 wherein the wound care device is adhesively connectable to the user.

6. The system of claim 1 wherein the disposible wound care device is a cushion configured to support a portion of the user's body

7. The system of any preceding claim, wherein said fluid supply is mounted to a bed.

8. The system of any preceding claim, wherein said fluid supply is a dedicated unit.

9. The system of any preceding claim, wherein said control interface is a bed controller.

10. The system of any one of claims 1 to 8, wherein said control interface is a dedicated unit.

11. The system of any preceding claim, further comprising a temperature sensor in communication with said control interface, said temperature sensor configured to indicate the temperature of fluid supplied by the fluid supply to said reservoir.

12. The system of any preceding claim, wherein said fluid supply is configured to control the temperature of air supplied to said reservoir.

13. The system of any preceding claim, further comprising at least one healing factor applied to said filter sheet.

14. The system of any preceding claim including a valve configured to adjustably meter the amount of fluid that is vented out of the wound care device.

15. The system of any preceding claim including a vent for allowing fluid to flow out of the reservoir.
